# EUROPEAN PATENT SPECIFICATION

(11) **EP 1 076 538 B1**
(45) Date of publication and mention of the grant of the patent: **08.10.2003**
(21) Application number: 99916813.1
(22) Date of filing: 03.05.1999
(51) Int. Cl.: A61F 2/48, A61F 5/48

(54) **A DEVICE FOR INSERTING INTO A BODY ORIFICE AND A METHOD FOR PRODUCING THIS DEVICE**
VORRICHTUNG ZUM EINSETZEN IN EINE KÖRPERÖFFNUNG SOWIE HERSTELLUNGSVERFAHREN DAFÜR
DISPOSITIF D'INSERTION DANS UN ORIFICE DU CORPS ET PROCEDE DE PRODUCTION DUDIT DISPOSITIF

(30) Priority: 04.05.1998 DK 61198
(43) Date of publication of application: 21.02.2001
(73) Proprietor: Codan Steritex ApS, 3060 Espergaerde (DK)
(72) Inventor: JENSEN, Hanne, DK-2400 Koebenhavn NV (DK); NIELSEN, Henrik, Lindeskov, DK-2765 Smoerum (DK); SVENNINGSEN, Irene, DK-3450 Alleroed (DK)
(74) Representative: Fleck, Thomas, Dr. Dipl.-Chem.
(86) International application number: DK9900241
(87) International publication number: WO99056678

(56) References cited:
- WO-A1-88/10106
- US-A- 3 834 389
- US-A- 4 108 180
- US-A- 4 979 947

## Description

### FIELD OF THE INVENTION

The present invention relates to a device for inserting into a body orifice, a method for producing this device and a method for securing a string in a device for insertion into a body orifice. The device comprises a body made of an elastic and moulded material which is to be placed in the body orifice and the body of the device is provided with a string which can be used when the body is to be removed from the orifice.

### BACKGROUND OF THE INVENTION

Various devices are designed to be placed in a body orifice for shorter periods of time, e.g. in the vagina for the purpose of absorbing fluids or for preventing incontinence by supporting the bladder neck, or in the anus or a stoma orifice for preventing faecal incontinence, Such devices are normally provided with means for facilitating the removal of the device. The means may be a finger grip formed as an integrated part of the device or it may be a string or similar protruding from the device and in some cases even projecting of the orifice and renders it convenient to grip and pull out the device.

A well-known method for securing a string in a removable body is by sewing but when the device is moulded the insertion of the string by sewing makes the process more expensive as compared to a process where a string is incorporated during moulding.

A simple way to incorporate the string during moulding is to let the string form an arch in order to let the string enter the device in one site and exit from the device at another site whereafter the two ends of the string are joined. This method is known from EP B1- 0 363 421 in connection with a device for controlling female urinary incontinence wherein the string enters and exits the body at the distal end as described in figure 2 of this document and the joined string is protruding from the vagina to allow easy removal. This positioning of the string assures that the string will not be pressed against the wall of the vagina by the body of the device during use.

The advantage of this known product is that when the string is in the correct position it requires an acceptably high extraction force to pull out the string from the body of the device, and the cost of production for this product is very low. The disadvantage of this method is that it is quite difficult to position the string correctly. When the string is not positioned correctly, the string does not necessarily show an acceptable extraction force and another problem is that if the string is placed to close to the walls of the body, the structure of the moulded material between the string and the surface can - depending on which material is used - change and this can result in the formation of hard spots which are perceptible for the user.

A way to avoid this problem is to carry the string through the moulding form and join the two ends together instead of making an arch through one side of the device but doing this will render it impossible to both lead the string into and out of the body through passages in the distal end surface and the string would, in use, be positioned between the walls of the body orifice and the body, at least when the body is of a compact form which means a form that is not to be folded when inserted in the body orifice. The user will be able to feel the string when such a compact device is placed in the body orifice and the efforts to make the surface and the structure of the device smooth and comfortable would be more or less in vain.

Another possibility is to carry the string through the moulding form from the distal end of the device to the proximal end and then cut of the proximal part of the string at the surface of the device. This production method resulted in very low costs but the force needed to detach the string from the device has turned up to be too low under extreme conditions and the material tends to become hart around the top (the proximal end) of the product where the string is cut of.

### BRIEF DESCRIPTION OF THE INVENTION

The invention relates to a device for insertion into a body orifice which device comprises a body having a distal and a proximal end and being made of an elastic and moulded material, and at least one string which is secured to the body during moulding.

Furthermore, the invention relates to a method for making a device for insertion into a body orifice which device comprises a body having a distal and a proximal end and being made of an elastic and moulded material, and at least one string which is secured to the body during moulding.

Still further, the invention relates to a method of securing a string in a device for insertion into a body orifice.

### DETAILED DESCRIPTION OF THE INVENTION

The object of this invention is to provide a device for insertion into a body orifice comprising a body made of an elastic and moulded material and at least one string which is secured to the body during moulding.

The invention relates to a device for insertion into a body orifice which device comprises a body having a distal and end and a proximal end and being made of an elastic and moulded material, and at least one first string which exits from the distal end of the body of the device and which is provided with holding means and wherein one string is secured to the body during moulding. The invention is characterised in that at least one second string exits the proximal end of the body of the device, and that these strings are connected inside the body or is constituted by only one string.

The device has a smooth and comfortable surface, is simple and inexpensive to produce and has at least one string so strongly secured to the body of the device that the string does not get detached even at extreme conditions. Also the fastening of the string inside the device does not prevent the device from being pressed to half size and inserted in an applicator.

The invention relates to a method for securing a string in a device for insertion into a body orifice which device comprises a body having a distal and a proximal end and being made of an elastic and moulded material, and at least one string which is secured to the body during moulding wherein a first string is provided with holding means and is placed in a mould for preparing the device in such a manner that it exits the distal part of the mould and filling the mould with the material forming the device embedding the string.

### BRIEF DESCRIPTION OF THE INVENTION

This object is obtained when means are formed or secured on at least one string which exits from the distal end of the body of the device, when at least one string exits the proximal end of the body of the device and these strings are connected or only is constituted by one string. The means can be a soft material secured to the string by e.g. sewing or it can be one or more loops formed on the string. A loop is defined as a closed circle of string enclosing an amount of material of the body of the device. Preferably the loop(s) can be secured on the string by a knot.

It is an advantage to use at least two strings as it is then possible to vary the thickness and the strength of the string(s) passing out of the distal and the proximal end of the device respectively. The string which exits the distal end has to be strong enough to resist the extraction force when the device is pulled out of the orifice. The string which exits the proximal end only has to be strong enough to keep the string(s) in position until the material has filled the moulding form.

Also a string with a small cross section passing out of the proximal end of the device does not leave a hard spot at the point where it passes through the surface of the body of the device. So in order to keep the surface of the proximal end of the device smooth and comfortable the string or strings passing through the proximal end of the body of the device can have a reduced total thickness, preferably a total thickness below 0,5·10⁻³ m or more preferred below 0,3·10⁻³ m. It appears that one or two threads with a total thickness as ordinary sewing thread is suitable.

The invention also relates to a method for making a device comprising a body made of an elastic moulded material and at least one string secured to the body which method comprises the following steps:
a) holding means are secured to or formed on at least one first string,
b) this string(s) alone or connected to one or more second string(s) is/are positioned in a moulding form where at least one first string exits the distal part of the moulding form and at least one second string exits the proximal end of the moulding form,
c) the raw materials are prepared and the moulding form is filled,
d) the material at least partly hardened,
e) the device is removed from the form,
f) the device is optionally dried.

Still further, the invention relates to a method for securing a string in a device for insertion into a body orifice which device comprises a body having a distal and a proximal end and being made of an elastic and moulded material, and at least one string which is secured to the body during moulding wherein a first string is provided with holding means and is placed in a mould for preparing the device in such a manner that it exits the distal part of the mould and filling the mould with the material forming the device embedding the string.

### BRIEF DESCRIPTION OF THE DRAWINGS

The invention is explained more in detail with reference to the drawings in which
Figure 1 shows a sectional view of a standard device not forming part of the invention,
Figure 2 shows a sectional view of a preferred embodiment of a device of the invention,
Figure 3 shows a sectional view of another preferred embodiment of a device of the invention, and
Figure 4 shows a graphical presentation of the extraction force of string from device for different devices.

### DETAILED DESCRIPTION OF THE DRAWINGS

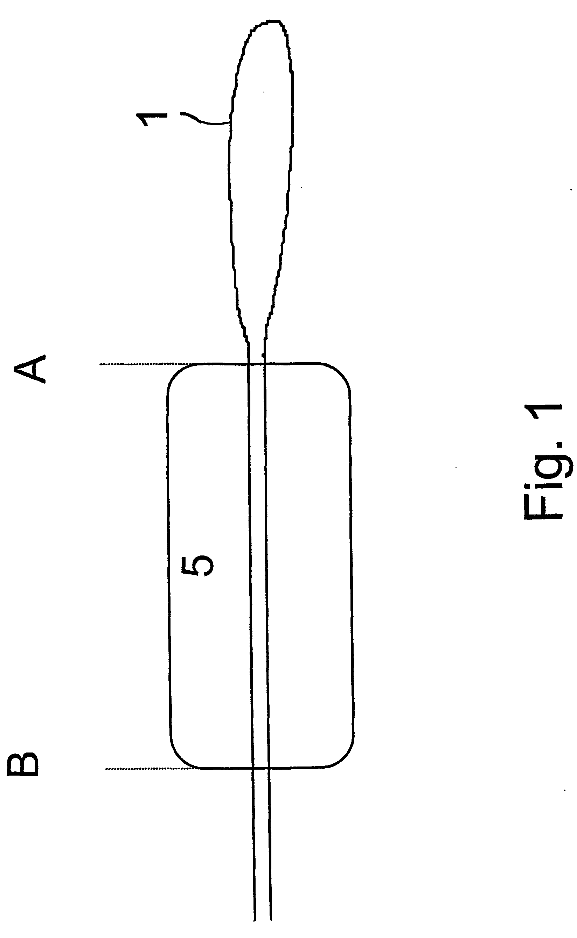

Figure 1 shows a sectional view of a standard state of the art device. This device comprises a string 1 and a body 5 made of a foam material for example as described in DK patent application no. 0014/98. The string is secured to the device during moulding and the double string 1 pass through both the distal end A and the proximal end B of the moulding form in order to assure the position of the string.

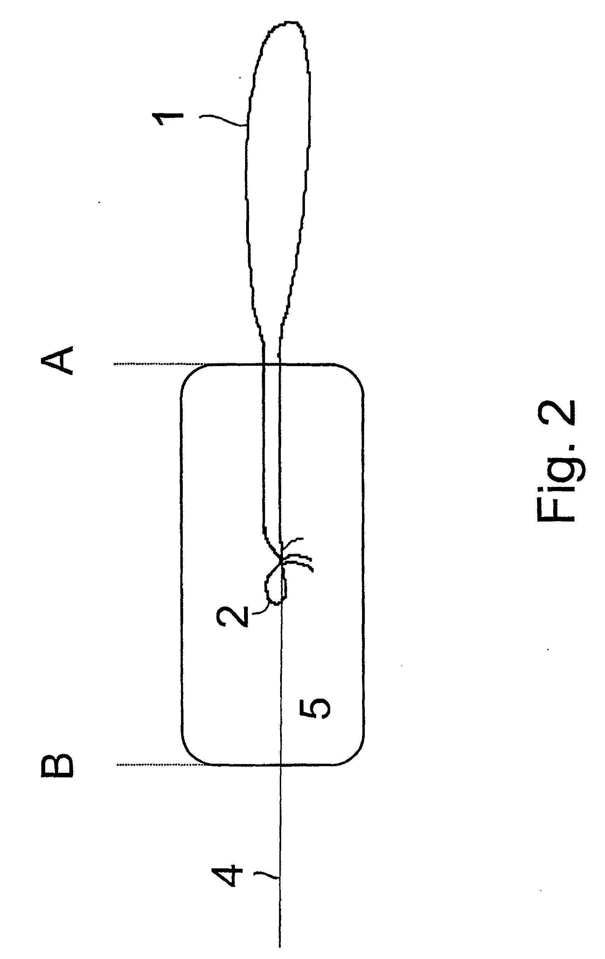

Figure 2 shows a sectional view of a device representing a preferred embodiment of the invention. This device comprises a string 1, a thread 4 (drawn in a thinner line) and a body 5. The string 1 is made with a loop 2 and the thread 4 is connected to the string 1. The thread can be connected to the string in many ways but a simple way is to tied the string and the thread together in a knot. The double string 1 and the thread has been placed in an elastic body 5.

This device has been manufactured according to the invention by forming a loop on a string and connecting the string to a thin thread. The connection between the string and the thread has been made by tying the string and the thread together in a knot and afterwards the double string and the long ends of the thread are separated and placed in a moulding form with the string stretched out of the distal end and the thread stretched out of the proximal end then they are secured in order to keep the position.

Then the moulding forms are filled with the elastic material and the material allowed at least partly to harden.

When the products has at least partly hardened they are removed from the moulding forms and dried. After drying the long thread at the proximal end is cut off right above the surface of the body of the product.

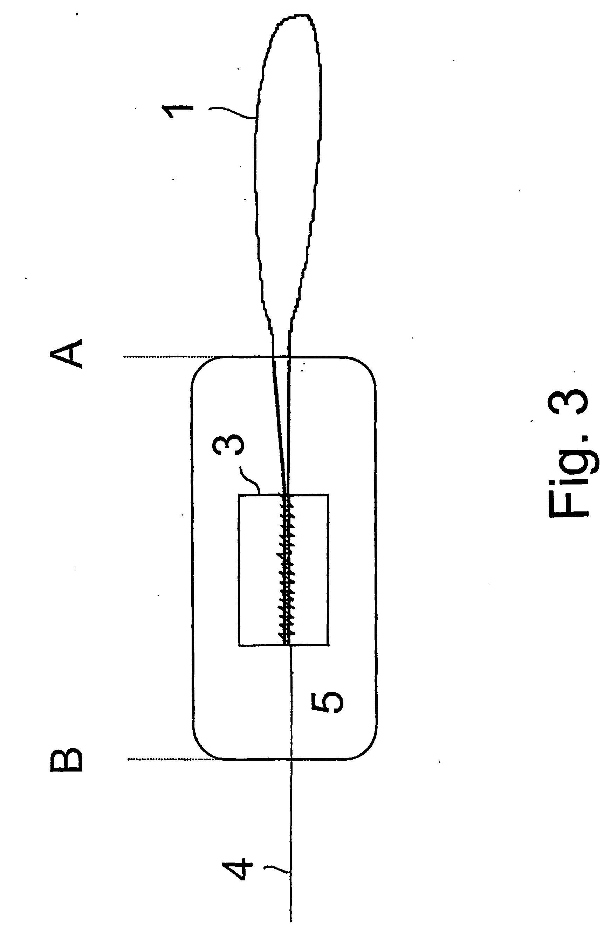

Reference is made to Figure 3 showing a sectional view of a device representing another preferred embodiment of the invention. This device comprises a double string 1, a thread 4 (drawn in a thinner line) a piece of soft tissue 3 and a body 5. The double string 1 is secured to the soft tissue 3 with the thread 4. The thread 4 is used to sew the double string 1 to the soft tissue 3. The double string 1, the thread 4 and the soft tissue 3 are embedded in an elastic body 5.

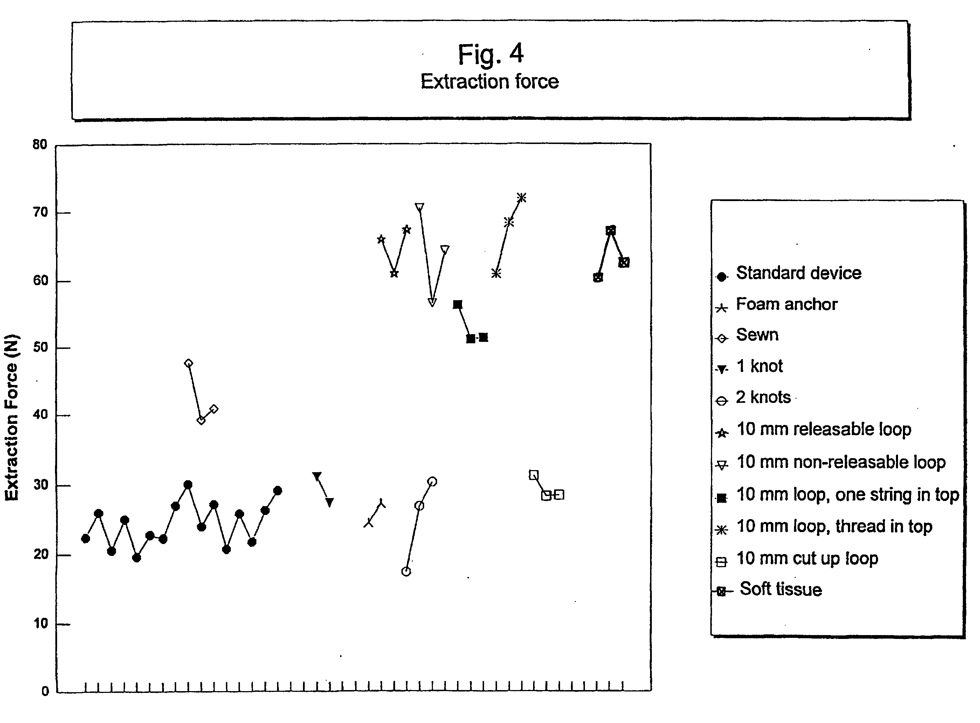

Figure 4 shows the extraction force needed to pull the string out of various devices. Experience has shown that for the string to be unreleasably secured to the body of the device even when the device has to be withdrawn from the body orifice under extreme conditions the extraction force needed to release the string from the body of the device should be above approximately 40 N.

### EXPERIMENTAL PART

Different embodiments of devices were tested in order to establish which embodiment had appropriate low production costs and at the same time had an extraction force for the string at least about 40 N.

### Experiment 1.

In the standard device shown in Fig. 1 a double string forms a loop outside the body of the device and the two strings runs parallel from the distal to the proximal end of the body where the strings are secured during moulding. The device was very economical to produce but it had an extraction force of only 18-30 N. In order to increase this extraction force the following ways of fastening the string to the body of the device was tried:

### Experiment 2.

A foam anchor of the same material as the body of the device was secured to a double string and afterwards the double string and the foam anchor was moulded into the body of the device. The device showed an extraction force for the string of about 24-28 N.

### Experiment 3.

The string was sewed into the body of the device using a needle drawing the string from the distal end to the proximal end of the device and back. The string of this device showed and extraction force of about 39-58 N.

### Experiment 4.

One knot was tied on a double string and afterwards the string was secured to the body of the device during moulding. The extraction force for the string of this device was about 27-32 N.

### Experiment 5.

Two knots were tied on a double string which was afterwards secured to the body of a device during moulding. The extraction force for the string of this device was about 18-31 N.

### Experiment 6.

A releasable loop of 10 mm was formed on the double string and afterwards the double string was secured to the body of the device during moulding. The extraction force for the string of this device was about 60-67 N.

### Experiment 7.

A non-releasable loop of 10 mm was formed on the double string and afterwards the double string was secured to the body of the device during moulding. The extraction force for the string of this device was about 55-70 N.

### Experiment 8.

A loop of 10 mm was formed on a double string and a single string was attached to the knot. Afterwards the strings were embedded in the body of the device during moulding with the double string protruding the distal end and the single string protruding the proximal end. The extraction force of the string of this device was about 52-57 N.

### Experiment 9.

A device as shown in figure 2 using a string with a loop of 10 mm. The extraction force of the string was about 60-72 N.

### Experiment 10.

A device as shown in figure 2 having a string wherein the loop has been cut up and no longer performs a closed circle was produced. The extraction force of the string was about 28-32 N.

It seems to be a significant parameter that part of the elastic moulded material is caught in the loop. What size the loop or several loops has to have depends on the moulded material and on the string forming the loop. A maximum and a minimum loop size has to be determined for every material.

### Experiment 11.

A sheet of soft tissue was secured to a double string as shown in figure 3 and afterwards the sheet and double string were embedded in the body of the device during moulding. The extraction force was about 60 - 67 N.

It seems that adhesion between the soft tissue and the body is significantly better than just to the string itself. The optimal size of the soft tissue has to be determined for every size and design of the body.

## Claims

1. A device for insertion into a body orifice which device comprises a body (5) having a distal and end (A) and a proximal end (B) and being made of an elastic and moulded material, and at least one first string (1) which exits from the distal end of the body of the device and which is provided with holding means (2, 3) and wherein one string is secured to the body during moulding **CHARACTERISED IN that** at least one second string (4) exits the proximal end (B) of the body of the device, and that these strings (1, 4) are connected inside the body (5) or is constituted by only one string.

2. A device according to claim 1 **CHARACTERISED IN that** at least two strings, a first string (1) and a second string (4), are placed in the body (5).

3. A device according to claim 2 **CHARACTERISED IN that** at least one first string (1) exits the body's distal end (A) and at least one second string (4) exits the body's proximal end (B) and the first string(s) (1) and the second string(s) (4) are connected.

4. A device according to claim 3 **CHARACTERISED IN that** the first string(s) (1) has a total thickness larger than 0,5·10⁻³ m preferably larger than 0,9·10⁻³ m, and that the second string(s) (4) has a total thickness of less than 0,5·10⁻³ m, preferably less than 0,3·10⁻³ m.

5. A device according to claim 1-4 **CHARACTERISED IN that** the means (3) comprises a layer of a material secured to a distal string (1).

6. A device according to claim 5 **CHARACTERISED IN that** the material is a soft non-woven.

7. A device according to claim 5 **CHARACTERISED IN that** the means (3) comprises one or more loops (2) formed on the string (1).

8. A device according to claim 7 **CHARACTERISED IN that** the length of the part of the string (1) forming a loop (2) is at least 6 times the thickness of the string (1), preferably at least 9 times the thickness of the string.

9. A method for making a device comprising a body (5) made of an elastic moulded material and at least one string (1) secured to the body (5) which method comprises the following steps:
a) holding means (2, 3) are secured to or formed on at least one first string (1),
b) this string(s) (1) alone or connected to one or more second string(s) (4) is/are positioned in a moulding form where at least one first string (1) exits the distal part of the mould and at least one second string (4) exits the proximal end of the moulding form,
c) the raw materials are prepared and the moulding form is filled,
d) the material at least partly hardened,
e) the device is removed from the form,
f) the device is optionally dried.

10. A method according to claim 9, **CHARACTERISED in that** the string(s) (4) which exit(s) the proximal end (B) of the body (5) of the device is(are) cut off at the surface of the device.

11. A method for making a device according to claim 9 or 10, **CHARACTERISED in that**
at least the first string(s) exiting the distal end and at least one second string exiting the proximal end are connected and positioned in the moulding form.

12. A method for making a device according to claim 11, **CHARACTERISED in that** at least a first string (1) and at least one second string (4) are tied together in such a way that at least the first string (1) forms a loop (2) and the at least two strings (1, 4) are connected to each other, whereafter the at least two strings (1, 4) are positioned in a moulding form where the first string (1) exits the distal part of the moulding form and the second string (4) exits the proximal end of the moulding form.

13. A method for making a device according to claim 11 or 12, **CHARACTERISED in that** the first string(s) (1) with at least one loop (2) has a minimum total thickness of 0,5·10⁻³ m, preferably of 0,9·10⁻³ m, and the second string(s) (4) has a maximum total thickness of 0,5·10⁻³ m, preferably 0,3·10⁻³ m.

14. A method for securing a string in a device for insertion into a body orifice which device comprises a body (5) having a distal end (A) and a proximal end (B) and being made of an elastic and moulded material, and at least one string (1) which is secured to the body (5) during moulding wherein a first string (1) is provided with holding means (2, 3) and the first string (1) alone or connected to at least one second string (4) is placed in a mould for preparing the device in such a manner that at least one first string (1) exits the distal part of the mould and at least one second string (4) exits the proximal end of the mould and filling the mould with the material forming the device embedding the string.

## Patentansprüche

1. Vorrichtung zum Einführen in eine Körperöffnung, welche Vorrichtung einen Korpus (5) mit einem distalen Ende (A) und einem proximalen Ende (B) aufweist und aus einem elastischen und in einer Form geformten Material besteht, und mit mindestens einem ersten Faden (1), der aus dem distalen Ende des Korpus der Vorrichtung austritt und welcher mit Haltemitteln (2, 3) versehen ist, und wobei ein Faden während des Formens an dem Korpus festgelegt ist,
**dadurch gekennzeichnet,**
**daß** wenigstens ein zweiter Faden (4) aus dem proximalen Ende (B) des Korpus der Vorrichtung austritt und daß diese Fäden (1, 4) im Innern des Korpus (5) verbunden oder durch lediglich einen Faden gebildet sind.

2. Vorrichtung nach Anspruch 1, **dadurch gekennzeichnet, daß** wenigstens zwei Fäden, ein erster Faden (1) und ein zweiter Faden (4), in dem Korpus (5) plaziert sind.

3. Vorrichtung nach Anspruch 2, **dadurch gekennzeichnet, daß** wenigstens ein erster Faden (1) aus dem distalen Ende (A) des Korpus austritt und wenigstens ein zweiter Faden (4) aus dem proximalen Ende (B) des Korpus austritt und daß der erste Faden / die ersten Fäden (1) und der zweite Faden /die zweiten Fäden (4) miteinander verbunden sind.

4. Vorrichtung nach Anspruch 3, **dadurch gekennzeichnet, daß** der erste Faden / die ersten Fäden (1) eine Gesamtstärke von größer als 0,5 * 10⁻³ m, vorzugsweise größer als 0,9*10⁻³ m, aufweist/aufweisen und daß der zweite Faden /die zweiten Fäden (4) eine Gesamtstärke von weniger als 0,5*10⁻³ m, vorzugsweise weniger als 0,3*10⁻³ m, aufweist/aufweisen.

5. Vorrichtung nach Anspruch 1 - 4, **dadurch gekennzeichnet, daß** die Mittel (3) eine Lage aus einem an einem distalen Faden (1) befestigten Material aufweisen.

6. Vorrichtung nach Anspruch 5, **dadurch gekennzeichnet, daß** das Material ein weicher Vliesstoff ist.

7. Vorrichtung nach Anspruch 5, **dadurch gekennzeichnet, daß** das Mittel (3) eine oder mehrere an dem Faden (1) gebildete Schlaufe(n) (2) aufweist.

8. Vorrichtung nach Anspruch 7, **dadurch gekennzeichnet, daß** die Länge des eine Schlaufe (2) bildenden Teils des Fadens (1) wenigstens das Sechsfache der Stärke des Fadens (1), vorzugsweise wenigstens das Neunfache der Stärke des Fadens, beträgt.

9. Verfahren zum Herstellen einer Vorrichtung, die einen aus einem elastischen, in einer Form geformten Material gefertigten Korpus (5) und wenigstens einen an dem Korpus (5) festgelegten Faden (1) enthält, welches Verfahren die folgenden Schritte aufweist:
a) Haltemittel (2, 3) werden an wenigstens einem ersten Faden (1) befestigt bzw. geformt,
b) der Faden / die Fäden (1) wird/werden allein oder verbunden mit einem oder mehreren zweiten Faden/Fäden (4) in einer Form positioniert, wobei wenigstens ein erster Faden (1) aus dem distalen Teil der Form austritt und wenigstens ein zweiter Faden (4) aus dem proximalen Ende der Form austritt,
c) die Rohmaterialien werden bereitet, und die Form wird befüllt,
d) das Material wird wenigstens teilweise erhärtet,
e) die Vorrichtung wird aus der Form entnommen,
f) die Vorrichtung wird optional getrocknet.

10. Verfahren nach Anspruch 9, **dadurch gekennzeichnet, daß** der Faden / die Fäden (4), welcher/welche aus dem proximalen Ende (B) des Korpus (5) der Vorrichtung austritt/austreten, an der Oberfläche der Vorrichtung abgeschnitten wird/werden.

11. Verfahren zum Herstellen einer Vorrichtung gemäß Anspruch 9 oder 10, **dadurch gekennzeichnet, daß** wenigstens der erste Faden / die ersten Fäden, der/die aus dem distalen Ende austritt/austreten und wenigstens ein zweiter, aus dem proximalen Ende austretender Faden miteinander verbunden und in der Form positioniert werden.

12. Verfahren zum Herstellen einer Vorrichtung nach Anspruch 11, **dadurch gekennzeichnet, daß** wenigstens ein erster Faden (1) und wenigstens ein zweiter Faden (4) in einer solchen Weise zusammengebunden sind, daß wenigstens der erste Faden (1) eine Schlaufe (2) bildet und die wenigstens zwei Fäden (1, 4) miteinander verbunden werden, wonach die wenigstens zwei Fäden (1, 4) in einer Form positioniert werden, wobei der erste Faden (1) aus dem distalen Teil der Form austritt und der zweite Faden (4) aus dem proximalen Ende der Form austritt.

13. Verfahren zum Herstellen einer Vorrichtung nach Anspruch 11 oder 12, **dadurch gekennzeichnet, daß** der erste Faden / die ersten Fäden (1) mit wenigstens einer Schlaufe (2) eine minimale Gesamtstärke von 0,5*10⁻³ m, vorzugsweise 0,9*10⁻³ m, aufweist/aufweisen und daß der zweite Faden /die zweiten Fäden (4) eine maximale Gesamtstärke von 0,5*10⁻³ m, vorzugsweise 0,3*10⁻³ m, aufweist/aufweisen.

14. Verfahren zum Festlegen eines Fadens in einer Vorrichtung zum Einführen in eine Körperöffnung, welche Vorrichtung einen Korpus (5) mit einem distalen Ende (A) und einem proximalen Ende (B), der aus einem elastischen und in einer Form geformten Material gefertigt ist, sowie wenigstens einen Faden (1), der an dem Korpus- (5) während des Formens festgelegt wird, aufweist, und wobei ein erster Faden (1) mit Haltemitteln (2, 3) versehen wird und der erste Faden (1) allein oder verbunden mit wenigstens einem zweiten Faden (4) in einer Form zum Bilden der Vorrichtung in einer solchen Weise plaziert wird, daß wenigstens ein erster Faden (1) aus dem distalen Teil der Form austritt und daß wenigstens ein zweiter Faden (4) aus dem proximalen Ende der Form austritt, und wobei die Form mit dem Material befüllt wird, wodurch die Vorrichtung gebildet und der Faden eingebettet werden.

## Revendications

1. Dispositif pour insertion dans un orifice corporel, lequel dispositif comprend un corps (5) présentant une extrémité distale (A) et une extrémité proximale (B) et étant composé d'une matière élastique et moulée, et au moins un premier cordon (1) qui sort de l'extrémité distale du corps du dispositif et qui est muni de moyens de retenue (2, 3) et dans lequel un cordon est fixé au corps au cours du moulage, **caractérisé en ce qu'**au moins un second cordon (4) sort de l'extrémité proximale (B) du corps du dispositif, et **en ce que** ces cordons (1, 4) sont reliés à l'intérieur du corps (5) ou sont constitués d'un seul cordon.

2. Dispositif selon la revendication 1, **caractérisé en ce qu'**au moins deux cordons, un premier cordon (1) et un second cordon (4), sont placés dans le corps (5).

3. Dispositif selon la revendication 2, **caractérisé en ce qu'**au moins un premier cordon (1) sort de l'extrémité distale (A) du corps et au moins un second cordon (4) sort de l'extrémité proximale (B) du corps et le(s) premier(s) cordon(s) (1) et le(s) second(s) cordon(s) (4) sont reliés.

4. Dispositif selon la revendication 3, **caractérisé en ce que** le(s) premier(s) cordon(s) (1) présente5nt) une épaisseur totale supérieure à 0,5.10⁻³ m, de préférence supérieure à 0,9.10⁻³ m, et **en ce que** le(s)second(s) cordon(s) (4) présente(nt) une épaisseur totale inférieure à 0,5.10⁻³ m, de préférence inférieure à 0,3.10⁻³ m.

5. Dispositif selon les revendications 1 à 4, **caractérisé en ce que** les moyens (3) comprennent une couche d'une matière fixée à un cordon distal (1).

6. Dispositif selon la revendication 5, **caractérisé en ce que** la matière est une matière non tissée douce.

7. Dispositif selon la revendication 5, **caractérisé en ce que** les moyens (3) comprennent une ou plusieurs boucles (2) formées sur le cordon (1).

8. Dispositif selon la revendication 7, **caractérisé en ce que** la longueur de la partie du cordon (1) formant une boucle (2) représente au moins 6 fois l'épaisseur du cordon (1), de préférence au moins 9 fois l'épaisseur du cordon.

9. Procédé de fabrication d'un dispositif comprenant un corps (5) composé d'une matière élastique moulée et au moins un cordon (1) fixé au corps (5) lequel procédé comprend les étapes suivantes :
a) des moyens de retenue (2, 3) sont fixés ou formés sur au moins un premier cordon (1),
b) ce(s) cordon(s) (1) seul(s) ou relié(s) à un ou plusieurs seconds cordons (4) est/sont positionné(s) dans une forme de moulage dans laquelle au moins un premier cordon (1) sort de la partie distale du moule et au moins un second cordon (4) sort de l'extrémité proximale de la forme de moulage,
c) les matières premières sont préparées et la forme de moulage est remplie,
d) la matière est durcie au moins partiellement,
e) le dispositif est retiré de la forme,
f) facultativement, le dispositif est séché.

10. Procédé selon la revendication 9, **caractérisé en ce que** le(s) cordon(s) (4) qui sort(ent) de l'extrémité proximale (B) du corps (5) du dispositif est(sont) découpé(s) à la surface du dispositif.

11. Procédé de fabrication d'un dispositif selon la revendication 9 ou 10, **caractérisé en ce que**
au moins le(s) premier(s) cordon(s) sortant de l'extrémité distale et au moins un second cordon sortant de l'extrémité proximale sont reliés et positionnés dans la forme de moulage.

12. Procédé de fabrication d'un dispositif selon la revendication 11, **caractérisé en ce qu'**au moins un premier cordon (1) et au moins un second cordon (4) sont liés ensemble d'une manière telle qu'au moins le premier cordon (1) forme une boucle (2) et les deux cordons (1, 4) au moins sont reliés l'un à l'autre, les deux cordons (1, 4) au moins sont ensuite positionnés dans une forme de moulage où le premier cordon (1) sort de la partie distale de la forme de moulage et le second cordon (4) sort de l'extrémité proximale de la forme de moulage.

13. Procédé de fabrication d'un dispositif selon la revendication 11 ou 12, **caractérisé en ce que** le(s) premier(s) cordon(s) (1) avec au moins une boucle (2) présente(nt) une épaisseur totale minimale de 0,5.10⁻³ m, de préférence de 0,9.10⁻³ m, et **en ce que** le(s) second(s) cordon(s) (4) présente(nt) une épaisseur totale maximale de 0,5.10⁻³ m, de préférence de 0,3.10⁻³ m.

14. Procédé pour fixer un cordon dans un dispositif pour insertion dans un orifice corporel lequel dispositif comprend un corps (5) présentant une extrémité distale (A) et une extrémité proximale (B) et étant composé d'une matière élastique et moulée, et au moins un cordon (1) qui est fixé au corps (5) au cours du moulage dans lequel un premier cordon (1) est muni de moyens de retenue (2, 3) et le premier cordon (1) seul ou relié à au moins un second cordon (4) est placé dans un moule pour préparer le dispositif d'une manière telle qu'au moins un premier cordon (1) sorte de la partie distale du moule et qu'au moins un second cordon (4) sorte de l'extrémité proximale du moule et pour remplir le moule d'une matière formant le dispositif intégrant le cordon.
